(19) Europäisches Patentamt
European Patent Office
Office européen des brevets

(11) Veröffentlichungsnummer: **0 130 514**
**A2**

(12) **EUROPÄISCHE PATENTANMELDUNG**

(21) Anmeldenummer: **84107247.3**

(22) Anmeldetag: **25.06.84**

(51) Int. Cl.⁴: **A 61 K 31/19**, A 61 K 31/60, A 61 K 47/00, A 61 K 31/33

(30) Priorität: **05.07.83 DE 3324193**

(43) Veröffentlichungstag der Anmeldung: **09.01.85 Patentblatt 85/2**

(84) Benannte Vertragsstaaten: **AT BE CH DE FR GB IT LI LU NL SE**


(71) Anmelder: **Troponwerke GmbH & Co. KG, Berliner Strasse 156, D-5000 Köln 80 (DE)**

(72) Erfinder: **Dell, Hans-Dieter, Dr., Kalmüntener Strasse 5, D-5060 Berg.-Gladbach 2 (DE)**
Erfinder: **Pelster, Bernhard, Dr., Pleisufer 6a, D-5205 St. Augustin 1 (DE)**
Erfinder: **Kraus, Reinhold, Paffendorfstrasse 77, D-5000 Köln 91 (DE)**
Erfinder: **Schierstedt, Detlef, Dr., Henri-Dumont-Strasse 2, D-5205 St. Augustin 3 (DE)**

(74) Vertreter: **Adrian, Albert, Dr. et al, c/o BAYER AG Konzernverwaltung RP Patentabteilung, D-5090 Leverkusen 1, Bayerwerk (DE)**


(54) **Depot-Antiphlogistika.**


(57) Die vorliegende Erfindung betrifft Verbindungen der allgemeinen Formel I

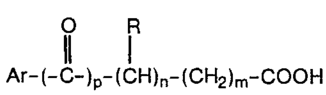

in welcher
R für Wasserstoff, niedriges Alkyl, substituiertes Alkyl,
Ar für Aryl, Heteroaryl, substituiertes Aryl, substituiertes Heteroaryl steht,
m und/oder n Null sein kann, bzw. n + m die Zahlen 1–2 sind und p Null oder 1 ist,
sowie deren Ester, Amide oder Salze,
in intramuskulär applizierbaren Arzneimitteln, insbesondere in solchen, die der Behandlung entzündlicher und/oder schmerzhafter Prozesse dienen und sich in dieser Form als Depotpräparate von den z.B. p.a. oder i.v. applizierten Präparaten durch ihre verlängerte Wirkungsdauer unterscheiden.


EP 0 130 514 A2

TROPONWERKE GmbH & Co. KG 5000 Köln 80
Je/ABc

# Depot-Antiphlogistika

Die vorliegende Erfindung betrifft Depotzubereitungen, enthaltend eine Verbindung der allgemeinen Formel I

$$Ar-(-\overset{O}{\overset{\|}{C}}-)_p-(\overset{R}{\overset{|}{C}}H)_n-CH_2)_m-COOH \qquad I$$

in welcher

R für Wasserstoff, niedriges Alkyl, substituiertes Alkyl,

Ar für Aryl, Heteroaryl, substituiertes Aryl, substituiertes Heteroaryl steht, und

m und/oder n Null sein kann, n + m die Zahlen 1-2 sind und p Null oder 1 ist,

sowie deren Ester, Amide oder Salze.

Antiphlogistisch-analgetisch Wirkstoffe werden bisher in oraler, rektaler, cutaner sowie intramuskulärer Applikationsweise bei Mensch und Tier angewendet. Hierbei ist die biologische Halbwertzeit und die Wirkdauer für das Applikationsschema ausschlaggebend. Bei zahlreichen dieser Medikamente muß eine tägliche Mehrfachapplikation erfolgen.

Zur Verlängerung der Wirkdauer sind zahlreiche Wirkstoffe in spezieller galenischer Verarbeitung als Retard-Präparate beschrieben, z.B. 1-(p-Chlorbenzoyl)-5-methoxy-2-methylindol-3-essigsäure als sustained release-Präparat in US-PS 4 173 626.

Es gibt bisher kein Antiphlogistikum/Analgetikum in Depot-Form; welches bei Applikation in mehrtägigen Abständen noch eine ausreichende Wirkung entfaltet.

Aufgabe der vorliegenden Erfindung war es daher, ein Antiphlogistikum/Analgetikum in Depot-Form bereitzustellen.

Der Erfindung liegt die Tatsache zugrunde, daß überraschenderweise gefunden wurde, daß Verbindungen der allgemeinen Formel I in geeigneter Form bei intramuskulärer Anwendung über einen wesentlich längeren Zeitraum stark entzündungshemmend wirken als die gleichen Wirkstoffe z.B. in oral zu verabreichender Form.

Gegenstand der vorliegenden Erfindung sind daher Mittel, enthaltend mindestens eine Verbindung der allgemeinen Formel I und ein Suspensionsmedium sowie ein

Verfahren zur Herstellung dieser Mittel und die Verwendung dieser Mittel als Depot-Antiphlogistikum bzw. Depot-Analgetikum.

Die Depotzubereitungen enthalten mindestens eine der Verbindungen der allgemeinen Formel I sowie ein Suspensionsmedium. Unter Suspensionmedium im Sinne der vorliegenden Erfindung werden verstanden:

Mono-, Di- und Triglyceride mit Mono-, Di- und Tricarbonsäuren der Kettenlänge $C_2$-$C_{30}$, in gesättigter wie ungesättigter, gegebenenfalls auch hydroxylierter Form (insbesondere ölige Triglyceride wie Viscoleo, Baumwollsaat-, Erdnuß-, Maiskeim-, Mandel-, Oliven-, Ricinus-, Sesamöl). Ferner sind geeignet Ester ein- oder mehrwertiger Alkohole, z.B. Propylenglykol, Butandiole und höhere Alkanole, Alkandiole, der Kettenlänge $C_2$-$C_{30}$ mit den oben erwähnten Säurekomponenten (als Beispiele seien genannt: Ethyloleat, Isopropylmyristat, Isopropylstearat), sowie gegebenenfalls die entsprechenden zuvor erwähnten Alkohole selbst.

Das Suspensionsmedium kann an Stelle oder neben geeigneten Estern auch Ether, Alkohole und Amide (gesättigt wie ungesättigt, aliphatisch, aromatisch) enthalten, die Amide können auch cyclisiert sein, z.B. Pyrrolidon-(2) (als Monomeres oder auch Polymeres). Diese können auch polyfunktionell sein, z.B. Polyole, Mono- und Diketale, Acetale, Polyether, cyclische Ether etc., welche toxikologisch indifferent sind.

Der Anteil des Suspensionsmediums in der erfindungsgemäßen Depotzubereitung liegt zwischen 2 und 90 %, vorzugsweise 3-70 %, besonders bevorzugt 10-60 Gew.-%.

Die sterilen Lösungen oder Suspensionen für Injektionszwecke können zusätzliche Geliermittel, z.B. Aluminiumstearat enthalten, um die Liberation aus dem Öldepot zu verzögern.

Gegebenenfalls können ein oder mehrere Konservierungsmittel verwendet werden, z.B. Benzylalkohol, Phenylethylalkohol, Chlorbutanol, Phenolderivate, z.B. Chlorkresol.

Gegebenenfalls werden dem Suspensionsmedium ein oder mehrere Antioxidantien zugesetzt, z.B. Tokopherole, Nordihydroguajaretsäure, Anisol-Derivate, Ascorbin-, Gallussäureester, Butylhydroxytoluole.

Das Depotpräparat kann gegebenenfalls auch in zunächst getrennter Form (Wirkstoff/Lösungs- bzw. Suspensionsmedium) vorliegen und vor Gebrauch vereinigt werden (z.B. Trockenampulle).

Unter Verbindungen der allgemeinen Formel I werden erfindungsgemäß verstanden:

$$Ar-(\overset{\overset{O}{\|}}{C}-)_p-(\overset{\overset{R}{|}}{C}H)_n-CH_2)_m-COOH \qquad (I)$$

in welcher

R für Wasserstoff, niedriges Alkyl, substituiertes Alkyl,

Ar für Aryl, Heteroaryl, substituiertes Aryl, substituiertes Heteroaryl steht, und

m und/oder n Null sein kann, n + m die Zahlen 1-2 sind und p Null oder 1 ist,

sowie deren Ester, Amide oder Salze.

Niedriges Alkyl R bedeutet R mit 1-6 C-Atomen, substituiertes Alkyl bedeutet Alkoxyalkyl, Trihalogenalkyl.

Aryl bzw. Heteroaryl bedeuten bevorzugt Phenyl, Naphthyl, Thiophenyl, Pyrrolyl, Indenyl, Indolyl, Benzthiazinyl, Phenothiazinyl.

Bevorzugt werden solche Verbindungen der allgemeinen Formel I verstanden, in welcher

R für H, Alkyl mit 1-4 C-Atomen, substituiertes Alkyl

m und/oder n Null sein kann,
n+m die Zahlen 1-2 sind und
p Null oder eins ist,

Ar für Aryl, Heteroaryl und deren substituierten Derivaten steht, wobei sich als Substituenten für Aryl

bzw. Heteroaryl bevorzugt Alkyl, bevorzugt grad- und verzweigtkettiges Alkyl mit bis zu 6 C-Atomen, Alkoxy, Oxalkyl, Acyl, Hydroxyl, Acetoxy, Benzoyl, substituiertes Benzoyl, Phenyl, substituiertes Phenyl, Phenoxy, Halogen, Phenylalkenyl eignen.

Neben den Säuren kommen deren Ester, Amide und Salze in Frage.

Die Ester sind Alkylester mit 1-6 C-Atomen, bevorzugt Methyl, Ethyl, i- und n-Propyl, substituiertes Alkyl, z.B. ß-Hydroxyethyl, Ester mit Glycolsäure. Die Amide können in der Gruppierung $-CO-NH_2$ an Stelle eines oder beider Amid-Wasserstoffe auch niedere Alkyle bzw. substituierte Alkyle enthalten.

Salze sind z.B. solche mit Alkali, Erdalkali, Aluminium, etc.

Ganz besonders bevorzugt werden unter den erfindungsgemäßen Depotzubereitungen solche verstanden, die als Wirkstoff mindestens eine der folgenden Verbindungen enthalten:

1. 2-Hydroxybenzoesäure 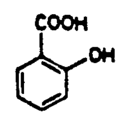

2. Acetoxybenzoesäure 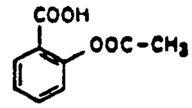

3. 2',4'-Difluor-4-hydroxy-3-biphenylcarbonsäure

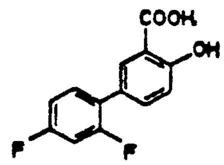

4. 2-Hydroxybenzamid

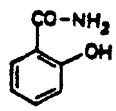

5. [2-(Aminocarbonyl)phenoxy]-essigsäure

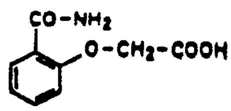

6. 4-Allyloxy-3-chlorphenyl-essigsäure

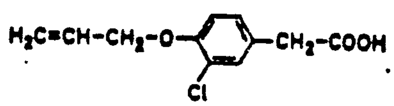

7. 2-[(2,6-Dichlorphenyl)-amino]-phenyl-essigsäure

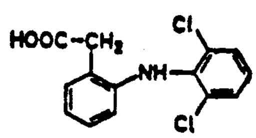

8. 10-Methyl-phenothiazin-2-yl-essigsäure

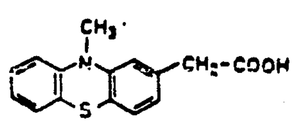

9. 1-Methyl-5-(p-toluoyl)-pyrrol-2-essigsäure

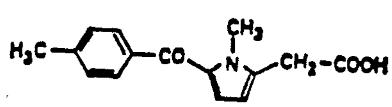

10. D-2-(6-Methoxy-2-naphthyl)-
propionsäure

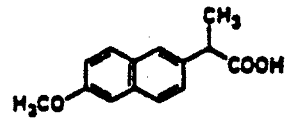

11. 2-(p-Isobutylphenyl)-propionsäure

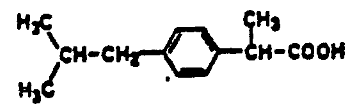

12. 2-(3-Phenoxyphenyl)-propionsäure

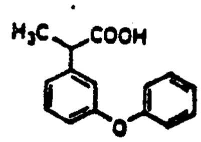

13. 2-(m-Benzoylphenyl)-propionsäure

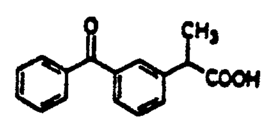

14. 2-[4-(1-Oxo-2-isoindolinyl)-
phenyl] -propionsäure

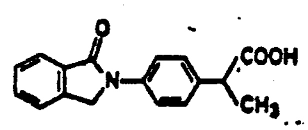

15. 2-(2-Fluorbiphenyl-4-yl)-
propionsäure

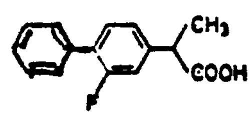

16. 3-(4-Biphenylylcarbonyl)-
propionsäure

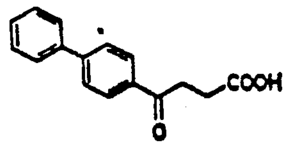

17. 2-(5-Benzoyl-2-thienyl)-
propionsäure

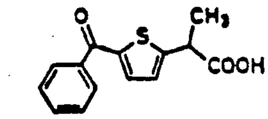

18. 1-(p-Chlorbenzoyl)-5-methoxy-
2-methylindol-3-essigsäure

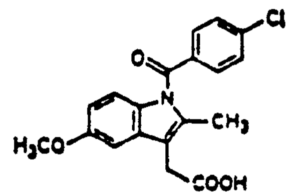

19. [1-(p-Chlorbenzoyl)-5-methoxy-2-
methylindol-3-acetoxy]essigsäure

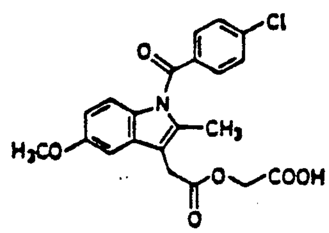

20. (Z)-5-Fluor-2-methyl-1-{[(4-
methylsulfinyl)phenyl]methylen}-1H-
inden-3-essigsäure

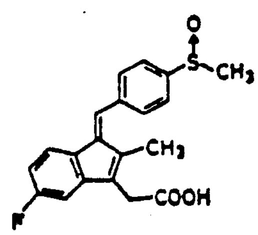

21. 4-Hydroxy-2-methyl-N-2-thiazolyl-
2H-1,2-benzothiazin-3-carboxamid
1,1-dioxid

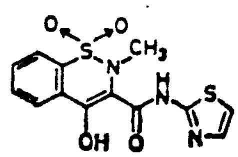

22. 4-Hydroxy-2-methyl-N-2-pyridinyl-2H-1,2-benzothiazin-3-carboxamid-1,1-dioxid

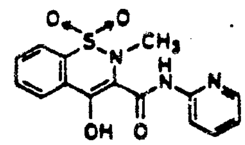

23. 4-Butyl-1,2-diphenyl-3,5-pyrazolidindion

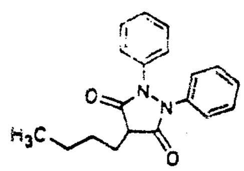

24. 4-n-Butyl-1-(p-hydroxyphenyl)-2-phenyl-pyrazolidindion

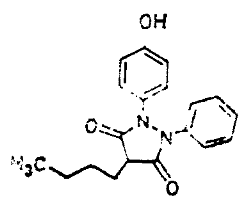

Gegebenenfalls können auch die Alkylester, Hydroxyalkylester und Hydroxyalkoxyalkylester dieser Säuren sowie substituierte Amide bzw. Salze eingesetzt werden.

Die pharmakologisch wirksamen Verbindungen der allgemeinen Formel I sind in dem erfindungsgemäßen Depotmittel in Mengen von 3 bis 80 Gew.-%, vorzugsweise von 5 bis 75 Gew.-%, besonders bevorzugt auf 10 bis 70 Gew.-% enthalten.

Ganz besonders bevorzugt enthalten die Depotzubereitungen als Suspensionsmedium mindestens eins aus der Gruppe:

Viscoleo, Isopropylmyristat, Ethyloleat, Ricinusöl, Sesamöl, Arachisöl, Baumwollsamenöl, Mandelöl, Olivenöl, Klauenöl, Neutralöl und Maisöl.

Unter Viscoleo wird ein Gemisch von Triglyceriden gesättigter Fettsäuren mittlerer Kettenlänge verstanden, das neutral und bei Zimmertemperatur flüssig ist. Insbesondere wird unter Viscoleo ein Gemisch verstanden, das der in Fiedler, "Lexikon der Hilfsstoffe für Pharmazie, Kosmetik und angrenzende Gebiete", 2. Auflage, 1981, Seite 993 gegebenen Definition entspricht.

Unter Klauenöl wird das aus den Klauen von Rindern, Hammeln oder den Füßen von Pferden durch Auskochen mit Wasser erhaltende, hellgelb gefärbte, nur wenig riechende Öl verstanden, entsprechend der Definition bei H.P. Fiedler "Lexikon der Hilfsstoffe für Pharmazie, Kosmetik und angrenzende Gebiete", 2. Aufl. Seite 790 (1981). Neutralöl, z. B. Miglyol, ist nach Fiedler (s.o., S. 615), Capryl-Caprinsäure-Triglycerid, eine Flüssigkeit mit niedriger Viskosität.

Die erfindungsgemäßen Depotmittel können außer den Wirkstoffen der allgemeinen Formel I noch weitere pharmazeutische Wirkstoffe enthalten.

Die Herstellung der vorstehend beschriebenen Zubereitungen erfolgt durch intensives Mischen des oder der Wirkstoffe der allgemeinen Formel I mit dem Suspensionsmedium in den vorstehend angegebenen Mengenverhältnissen.

Die Herstellung von Lösungen erfolgt üblicherweise durch Lösung der keimarmen bzw. sterilen Wirk- und Hilfsstoffe

im sterilisierten Lösungsmittel. Anschließend wird steril filtriert und in Ampullen abgefüllt.

Die Suspensionen werden aus sterilen Wirk- und Hilfsstoffen unter aseptischen Bedingungen bereitet. Gegebenenfalls wird (bei Lösung wie Suspension) unter Schutzgas gearbeitet, Hitzesterilisation ist in besonderen Fällen möglich.

Die Ampullenfüllung beträgt 0,5-5,0 ml. Die zu applizierende Lösung bzw. Suspension beträgt bevorzugt 1-3 ml, besonders bevorzugt 1-2 ml.

Die ödemhemmende und die analgetische Wirkung der Testsubstanzen nach i.m. Applikation einer entsprechenden Depot-Zubereitung wurde mit Hilfe des durch Carrageenan induzierten Ödems der Rattenpfote bzw. mittels des Randall-Selitto-Test /Arch. int. Pharmacodyn. 111, 409 (1957)/ ermittelt.

Die Versuche wurden mit männlichen Ratten /Stamm: Bor: WISW (SPF-Cpb), Gewicht um 200 g/ durchgeführt. Den Tieren wurde einmalig ein Depotpräparat i.m. appliziert und an den folgenden Tagen die entzündungshemmende Wirkung durch Auslösung des Carrageenan-Ödems überprüft. Pro Tag wurden jeweils drei Gruppen von je 5 Tieren untersucht, wobei die erste Gruppe aus 5 unbehandelten, die 2. Gruppe aus 5 nur mit Lösungsmittel (z.B. Viscoleo) behandelten und die 3. Gruppe aus 5 mit Depot-Präparat (z.B. 4-Hydroxy-2-methyl-N-2-pyridinyl-2H-1,2-benzothiazin-3-carboxamid-1,1-dioxid, Formel 22, in Viscoleo) behandelten Tieren bestand.

Die Pfotenvolumen wurden nach der Methode von F. Kemper und G. Ameln /Z. ges. exp. Med. 131, 407 (1959)7 gemessen, wobei die Differenz von Pfotenvolumen 5 Stunden nach der Ödemprovokation und dem normalen Pfotenvolumen das Ödemvolumen ergibt.

Die Hemmung des Carrageenan-Ödems nach i.m. Applikation verschiedener Wirkstoffe als Lösung oder Suspension in öligem Medium wurde 2 und 4 Tage post injectionem ermittelt. Die Applikation erfolgte mit unterschiedlichen Dosen bei konstantem Applikationsvolumen von 0,03 ml pro Ratte (siehe Tabelle).

| Substanz (mg/kg) | % Ödemhemmung 2. | 4. Tag p. inj. |
|---|---|---|
| 1-(p-Chlorbenzoyl)-5-methoxy-2-methylindol-3-essigsäure (18) 10,0 mg/kg), Suspension | 58,2 | 29,5 |
| 2',4'-Difluor-4-hydroxy-3-biphenylcarbonsäure (3) (5 mg/kg), Suspension | 26 | 13 |
| 2-(p-Isobutylphenyl)-propionsäure (11) (13 mg/kg), Suspension | 40 | 45 |
| D-2-(6-Methoxy-2-naphthyl)-propionsäure (10) (2,5 mg/kg), Suspension | 42,8 | 22,3 |
| 4-Hydroxy-2-methyl-N-2-pyridinyl-2H-1,2-benzothiazin-3-carboxamid-1,1-dioxid (22) (0,6 mg/kg), Suspension | 33 | 39 |
| 4-Hydroxy-2-methyl-N-2-thiazolyl-2H-1,2-benzothiazin-4-carboxamid-1,1-dioxid (21) (15 mg/kg), Suspension | 42,9 | 30,4 |

TP 54

(Fortsetzung)

| Substanz (mg/kg) | % Ödemhemmung 2. | 4. Tag p. inj. |
|---|---|---|
| 1-(p-Chlorbenzoyl)-5-methoxy-2-methylindol-3-essigsäure (18) (10,0mg/kg), Lösung | 58.2 | 29.5 |
| 4-Hydroxy-2-methyl-N-2-pyridinyl-2H-1,2-benzothiazin-3-carboxamid-1,1-dioxid (22) (0,6 mg/kg), Lösung | 33.2 | 39.3 |

P.o.-Applikation dieser Wirkstoffe führt beim gleichen Modell (Rattenpfotenödem) zu starker Ödemhemmung /H. Jacobi et al., Arzneimittelforsch. 27 1328 (1977) und 30, 1326 (1980)7. Diese liegt jedoch nach p.o.-Gabe, im Gegensatz zu den erfindungsgemäßen Depot-Präparationen, am 2. Tag post applicationem im Bereich der Kontrollwerte.

Im Gegensatz zur Verlängerung der Wirkung nach i.m.-Applikationen der erfindungsgemäßen Depot-Präparationen zeigt der Vergleich der biologischen Halbwertszeiten von Antiphlogistika nach p.o.-Gabe und i.m.-Applikation wäßriger Lösung, daß im letzteren Fall kein Unterschied in der Halbwertszeit besteht.

So beschrieben T. Ishizaki et al., (Eur. J. Clin. Pharmacol., 18, 407-414 (1980)), daß die biologische Halbwertszeit der Verbindung 13 beim Menschen nach p.o.-Gabe 1,13±0,07 Std., nach i.m.-Gabe wäßriger Lösung 1,27±

0,04 Std. beträgt; auch der maximale Plasmaspiegel, Plasma- und renale Clearance unterscheiden sich nicht.

Zur vorliegenden Erfindung gehört auch die Verwendung der Depot-Präparate in der Human- und Veterinärmedizin bei der Bekämpfung von entzündlichen und/oder schmerzhaften Prozessen.

Im allgemeinen empfiehlt es sich sowohl in der Humanals auch in der Veterinärmedizin, die erfindungsgemäßen Wirkstoffe in Gesamtmengen von etwa 0,1 bis etwa 100, vorzugsweise 0,3 bis 10 mg/kg Körpergewicht je Injektion zu verabreichen. Es kann jedoch erforderlich sein, von den genannten Dosierungen abzuweichen und zwar in Abhängigkeit von der Art und dem Körpergewicht des zu behandelnden Objektes und der Art und der Schwere der Erkrankungen.

Die folgenden Beispiele veranschaulichen erfindungsgemäße, injizierbare, ölige Lösungen bzw. Suspensionen, die als Depot-Antiphlogistika bzw. Depot-Analgetika eingesetzt werden können und besonders bevorzugte Zusammensetzungen darstellen:

Tab. 1: Beispiele von Suspensionen

| | | |
|---|---|---|
| 1 | 1-(p-Chlorbenzoyl)-5-methoxy-2-methylindol-3-essigsäure (Formel 18)<br>Isopropylmyristat | 200 g<br>ad 1000 g |
| 2 | /1-(p-Chlorbenzoyl)-5-methoxy-2-methylindol-3-acetoxy/essigsäure, Formel 19,<br>Isopropylmyristat | 200 g<br>ad 1000 g |
| 3 | 4-Hydroxy-2-methyl-N-2-pyridinyl-2H-1,2-benzothiazin-3-carboxamid-1,1-dioxid, Formel 22,<br>Aluminiumstearat<br>Viscoleo | 30 g<br>20 g<br>ad 1000 g |
| 4 | 4-Hydroxy-2-methyl-N-2-thiazolyl-2H-1,2-benzothiazin-3-carboxamid-1,1-dioxid, Formel 21<br>Aluminiumstearat<br>Viscoleo | 30 g<br>20 g<br>ad 1000 g |
| 5 | (Z)-5-Fluor-2-methyl-1-{/(4-methylsulfinyl)phenyl/methylen}-1H-inden-3-essigsäure, Formel 20<br>Miglyol 812 | 200 g<br>ad 1000 g |
| 6 | 2-(m-Benzoylphenyl)-propionsäure, Formel 13<br>Miglyol 812 | 250 g<br>ad 1000 g |
| 7 | 2-(2-Fluorbiphenyl-4-yl)-propionsäure, Formel 15<br>Gallussäurepropylester<br>Sesamöl | 250 g<br>10 g<br>ad 1000 g |

Tab. 2: Beispiele von öligen Lösungen

| | | |
|---|---|---|
| 1 | 4-Hydroxy-2-methyl-N-2-pyridinyl-2H-1,2-benzothiazin-3-carboxamid-1,1-dioxid, Formel 22 | 13,0 g |
| | Laktonal | 131,5 g |
| | Pyrrolidon-(2) | 255,5 g |
| | Miglyol 840 | ad 1000,0 g |
| 2 | 1-(p-Chlorbenzoyl)-5-methoxy-2-methylindol-3-essigsäure, Formel 18 | 150,0 g |
| | Laktonal | 112,0 g |
| | Pyrrolidon-(2) | 300,0 g |
| | Miglyol 840 | ad 1000,0 g |
| 3 | 2-(m-Benzoylphenyl)-propionsäure, Formel 13, | 100,0 g |
| | Laktonal | 90,0 g |
| | Pyrrolidon-(2) | 250,0 g |
| | Miglyol 840 | ad 1000,0 g |

Unter Miglyol 840 werden die Propylenglykolderivate der Capryl- und Capronsäure verstanden.

Die Herstellung der Lösungen erfolgt üblicherweise durch Lösung der keimarmen bzw. sterilen Wirk- und Hilfsstoffe im sterilisierten Lösungsmittel. Anschließend wird steril filtriert und in Ampullen abgefüllt.

Die Suspensionen werden aus sterilen Wirk- und Hilfsstoffen unte aseptischen Bedingungen bereitet. Gegebenenfalls wird (bei Lösung wie Suspension) unter Schutzgas gearbeitet, Hitzesterilisation ist in besonderen Fällen möglich.

| | | |
|---|---|---|
| Arachisöl | = | Ol. arachidis neutralisatum |
| Baumwollsamenöl | = | Ol. gossypii " |
| Mandelöl | = | Ol. amygdalae " |
| Olivenöl | = | Ol olivae " |
| Sesamöl | = | Ol. sesami " |
| Ricinusöl | = | Ol. ricini " |
| Klauenöl | = | Ol. pedis bovis/equi |
| Neutralöl | = | Ol neutrale (z.B. Miglyol) |
| Maisöl | = | Ol. maydis neutralisatum |

Die vorliegende Erfindung soll durch die nachfolgenden Beispiele noch näher erläutert werden.

Tabelle 3 (ölige Lösungen von D-2-(6-Methoxy-2-Naphthyl) propionsäure)

| Beispiel | /mg/ | Lösungs- bzw. Suspensions-medium /ad 1000 mg/ | Gallussäure-propylester /mg/ | Al-Stearat /mg/ | Neutralöl /mg/ |
|---|---|---|---|---|---|
| 11 | 270 | Arachisöl | 10 | | |
| 12 | 100 | Arachisöl | 10 | | |
| 13 | 260 | Baumwollsamenöl | | | |
| 14 | 100 | Baumwollsamenöl | | 20 | |
| 15 | 260 | Mandelöl | | | |
| 16 | 100 | Mandelöl | | | |
| 17 | 270 | Maisöl | | | |
| 18 | 100 | Maisöl | | | |
| 19 | 280 | Olivenöl | 10 | | |
| 20 | 100 | Olivenöl | 10 | | |
| 21 | 250 | Rizinusöl | 10 | | |
| 22 | 100 | Rizinusöl | 10 | | |
| 23 | 260 | Sesamöl | | | |
| 24 | 100 | Sesamöl | | | |
| 25 | 270 | Klauenöl | | | |
| 26 | 100 | Klauenöl | | 20 | |
| 27 | 280 | Neutralöl | | | |
| 28 | 100 | Neutralöl | | 20 | |
| 29 | 280 | Ethyloleat | | | |
| 30 | 100 | Ethyloleat | | | |
| 31 | 280 | Isopropymyristat | | | |
| 32 | 100 | Isopropymyristat | | 20 | |
| 33 | 280 | Isopropylpalmitat | | | |
| 34 | 100 | Isopropylpamitat | | | |
| 35 | 250 | Oleyloleat | | | |

| Beispiel | /mg/ | Lösungs- bzw. Suspensionsmedium /ad 1000 mg/ | Gallussäurepropylester /mg/ | Al-Stearat /mg/ | Neutralöl /mg/ |
|---|---|---|---|---|---|
| 36 | 100 | Oleyloleat | | | |
| 37 | 250 | Benzylbenzoat | | | 250 |
| 38 | 90 | Benzylbenzoat | | | 500 |
| 39 | 250 | Mohnöl | 10 | | |
| 40 | 90 | Mohnöl | 10 | | |
| 41 | 260 | Viscoleo | | | |
| 42 | 100 | Viscoleo | | 20 | |

Analog wie in der Tabelle 3 wurden jeweils als Wirkstoff auch die folgenden Verbindungen eingesetzt:

13 2-(m-Benzoylphenyl)-propionsäure
15 2-(2-Fluorbiphenyl-4-yl)-propionsäure
17 2-(5-Benzoyl-2-thienyl)-propionsäure
18 1-(p-Chlorbenzoyl)-5-methoxy-2-methylindol-3-essigsäure
19 [1-(p-Chlorbenzoyl)-5-methoxy-2-methylindol-3-acetoxy]essigsäure
20 (Z)-5-Fluor-2-methyl-1-{[(4-methylsulfinyl)phenyl]methylen}-1H-inden-3-essigsäure
21 4-Hydroxy-2-methyl-N-2-pyridinyl-2H-1,2-benzothiazin-3-carboxamid-1,1-dioxid
22 4-Hydroxy-2-methyl-N-2-thiazolyl-2H-1,2-benzothiazin-3-carboxamid-1,1-dioxid
23 4-Butyl-1,2-diphenyl-3,5-pyrazolidindion
24 4-n-Butyl-1-(p-hydroxyphenyl)-2-phenyl-pyrazolidindion.

Patentansprüche

1. Depotmittel, enthaltend eine Verbindung der allgemeinen Formel (I)

$$Ar-(-\overset{O}{\overset{\|}{C}}-)_p-(\overset{R}{\overset{|}{C}H})_n-(CH_2)_m-COOH \qquad (I),$$

in welcher

R für Wasserstoff, niedriges Alkyl, substituiertes Alkyl,

Ar für Aryl, Heteroaryl, substituiertes Aryl, substituiertes Heteroaryl steht,

m + n zusammen 1 oder 2 ergeben,

m und/oder n Null sein kann und

p Null oder 1 ist,

sowie deren Ester, Amide oder Salze sowie ein Suspensionsmedium.

2. Depotmittel, enthaltend eine Verbindung der allgemeinen Formel (I) nach Anspruch 1, in welcher

R für Wasserstoff, Alkyl mit 1-4 C-Atomen, substituiertes Alkyl,

Ar für Aryl und Heteroaryl und deren substituierte Derivate, wobei als Substituenten für Aryl bzw. Heteroaryl Alkyl, bevorzugt mit 1-6 C-Atomen, Alkoxy, Oxalkyl, Acyl, Hydroxyl, Acetoxy, Benzoyl, substituiertes Benzoyl, Phenyl, substituiertes Phenyl, Phenoxy, Halogen, vorzugsweise Fluor, Chlor oder Brom, Phenylalkenyl steht und

m und n zusammen 1 oder 2 ergeben,

m und/oder n Null sein kann und

p Null oder 1 ist,

sowie deren Ester, Amide oder Salze sowie ein Suspensionsmedium.

3. Depotmittel, enthaltend ein Suspensionsmittel sowie mindestens eine Verbindung aus der Gruppe:

2-Hydroxybenzoesäure (1), 2-Acetoxybenzoesäure (2) 2',4'-Difluor-4-hydroxy-3-biphenylcarbonsäure (3), 2-Hydroxybenzamid (4), [2-(Aminocarbonyl)phenoxy]-essigsäure (5), 4-Allyloxy-3-chlorphenylessigsäure (6), 2-[(2,6-Dichlorphenyl)amino]-phenylessigsäure (7), 10-Methyl-phenothiazin-2-yl-essigsäure (8), 1-Methyl-5-(p-toluoyl)-pyrrol-2-essigsäure (9), D-2-(6-Methoxy-2-naphthyl)-propionsäure (10), 2-(p-Isobutylphenyl)-propionsäure (11), 2-(3-Phenoxyphenyl)-propionsäure (12),

2-(m-Benzoylphenyl)-propionsäure (13), 2-/4-(1-Oxo-2-isoindolinyl)-phenyl7-propionsäure (14), 2-(2-Fluorbiphenyl-4-yl)-propionsäure (15), 3-(4-Biphenylylcarbonyl)-propionsäure (16), 2-(5-Benzoyl-2-thienyl)-propionsäure (17), 1-(p-Chlorbenzoyl)-5-methoxy-2-methylindol-3-essigsäure (18), /1-(p-Chlorbenzoyl)-5-methoxy-2-methylindol-3-acetoxy7essigsäure (19), (Z)-5-Fluor-2-methyl-1-{/(4-methylsulfinyl)phenyl7methylen} -1H-inden-3-essigsäure (20), 4-Hydroxy-2-methyl-N-2-thiazolyl-2H-1,2-benzothiazin-3-carboxamid-1,1-dioxid (21), 4-Hydroxy-2-methyl-N-2-pyridinyl-2H-1,2-benzothiazin-3-carboxamid-1,1-dioxid (22), 4-Butyl-1,2-diphenyl-3,5-Pyrazolidindion(23), 4-n-Butyl-1-(p-hydroxyphenyl)-2-phenyl-pyrazolidindion (24).

4. Depotmittel nach einem oder mehreren der Ansprüche 1 bis 3, dadurch gekennzeichnet, daß es als Suspensionsmittel eines oder mehrere aus der Gruppe:

Viscoleo, Isopropylmyristat, Ethyloleat, Ricinusöl, Sesamöl, Arachisöl, Baumwollsaatöl, Mandelöl, Olivenöl, Klauenöl, Neutralöl und Maisöl enthält.

5. Depotmittel nach einem oder mehreren der Ansprüche 1 bis 3, dadurch gekennzeichnet, daß es als Suspensionsmittel eines oder mehrere aus der Gruppe:

Viscoleo, Isopropylmyristat, Ethyloleat, Neutralöl enthält.

6. Depotmittel nach einem oder mehreren der Ansprüche 1 bis 5, dadurch gekennzeichnet, daß es eine Verbindung der allgemeinen Formel (I) gemäß einem oder mehreren der Ansprüche 1 bis 3 in einer Menge von 3 bis 80 Gew.-%, vorzugsweise 10 bis 75 Gew.-% und ein Suspensionsmittel gemäß Anspruch 4 oder 5 in einer Menge von 5 bis 90 Gew.-%, vorzugsweise 20-80 Gew.-%, enthält.

7. Depotmittel nach einem oder mehreren der Ansprüche 1 bis 6, dadurch gekennzeichnet, daß es Konservierungsmittel und Antioxydantien enthält.

8. Verwendung von einem Depotmittel gemäß einem oder mehreren der Ansprüche 1 bis 7 zur Bekämpfung von entzündlichen und/oder schmerzhaften Prozessen in der Human- und Veterinärmedizin.

9. Verfahren zur Herstellung von Depotmitteln gemäß einem oder mehreren der Ansprüche 1 bis 8, dadurch gekennzeichnet, daß man eine Verbindung der allgemeinen Formel (I) gemäß einem oder mehreren der Ansprüche 1 bis 3 mit einem Suspensionsmittel gemäß Anspruch 4 oder 5 intensiv mischt und abfüllt.

10. Verfahren zur Herstellung von Depotmitteln gemäß einem oder mehreren der Ansprüche 1 bis 7, dadurch gekennzeichnet, daß man eine Verbindung der allgemeinen Formel (I) gemäß einem oder mehreren der Ansprüche 1 bis 3 mit einem Suspensionsmittel gemäß Anspruch 4 oder 5 in einer solchen Weise mischt, daß im fertigen Mittel 3 bis 80 Gew.-% der Verbindung der allgemeinen Formel (I) und 10 bis 70 Gew.-% des Suspensionsmittels enthalten sind.